# EUROPEAN PATENT APPLICATION

(11) **EP 1 847 526 A1**
(43) Date of publication of application: **24.10.2007**
(21) Application number: 06007949.8
(22) Date of filing: 18.04.2006
(51) Int. Cl.: C07C 215/80, C07C 215/84, C07C 217/90, C07C 323/34, C07C 323/36, C07C 323/37, A61Q 5/10, A61K 8/41, A61K 8/46

(54) **Coupling compounds and hair dyeing compositions containg them**

(71) Applicant: DyStar Textilfarben GmbH & Co. Deutschland KG, 65926 Frankfurt am Main (DE); Kao Corporation, Tokyo 131-8501 (JP)
(72) Inventor: Kühlwein, Jürgen, 63150 Heusenstamm (DE); Russ, Werner, 65439 Flörsheim-Wicker (DE); Pratt, Dominic, 64572 Büttelborn (DE); Möhring, Hartmut, 64342 Seeheim-Jugenheim (DE)
(74) Representative: Muley, Ralf

(57) **Abstract**

The present invention refers to Compound of the formula (I) 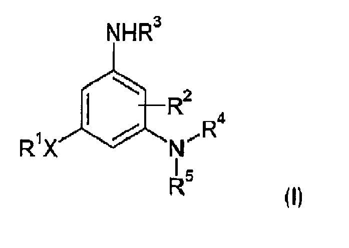 wherein
X
is oxygen or sulphur;
R¹
is (C₁-C₄)-alkyl, (C₁-C₄)-alkyl which is substituted by hydroxy, (C₁-C₄)-alkoxy, hydroxy-(C₁-C₄)-alkoxy, CN, -COOR⁶, -CON(R⁶)₂ or -N(R⁶)₂ or is phenyl;
R²
is hydrogen, methyl or ethyl;
R³
is hydrogen, (C₁-C₄)-alkylsulfonyl, (C₁-C₄)-alkylsulfonyl which is substituted by hydroxy, halogen, cyano, (C₁-C₄)-alkoxy or -N(R⁶)₂ or is -SO₂-CH=CH₂, -SO₂N(R⁶)₂ or -PO(OR⁶)₂; or
R³
is hydroxy-(C₁-C₄)-alkyl if R⁴ and R⁵ are not hydrogen ;
R⁴
is hydrogen, (C₁-C₄)-alkyl, (C₁-C₄)-alkyl which is substituted by hydroxy, (C₁-C₄)-alkoxy, or hydroxy-(C₁-C₄)-alkoxy ;
R⁵
is hydrogen, (C₁-C₄)-alkyl, (C₁-C₄)-alkyl which is substituted by hydroxy, (C₁-C₄)-alkoxy or hydroxy-(C₁-C₄)-alkoxy ;
R⁶
is hydrogen, (C₁-C₄)-alkyl or hydroxy-(C₁-C₄)-alkyl;

whereas R³, R⁴ and R⁶ are all hydrogen or are different and whereas the compound of the formula (I) wherein R¹ is methyl and R² to R⁵ are hydrogen is diesclaimt, as well as hair dye compositions containing them.

## Description

The present invention relates to novel coupling compounds and hair dye compositions containing them.

It is known to colour human hair with dyeing compositions containing oxidation dye precursors, also called oxidation bases or developers.
Oxidation bases are colourless or weakly coloured compounds, such as ortho- or para-phenylenediamines, ortho- or para-aminophenols or heterocyclic compounds and react with oxidizing agents to give coloured compounds.
The obtained shades can be varied by combining the oxidation bases with couplers, such as aromatic meta-diamines, meta-aminophenols, metahydroxyphenols and certain heterocyclic compounds. A variety of oxidation bases and couplers can be used to enable to get a broad range of different shades.
The permanent colours obtained by using these oxidation dyes need to fulfil some requirements. They should be safe, provide good intensity evenly along the hair shaft, should be stable to external influences, such as shampooing, light, sweat and rubbing and should enable the coverage of grey hair. The dyes should also be stable in the formulation.
There is a constant need to get new developers and couplers that improve at least one of these requirements, particularly there is a constant need to find new couplers which provide improved colour properties

m-Phenylendiamines and their use coupling compounds to produce oxidation dyes for hair dyeing are known and for example described in DE 35 21 995 A1, WO1988/00042, WO1993/010744, DE 102 60 834 A1 and WO2004/058204.

It was now surprisingly found that specific compounds according to the definition given below can be used as couplers and fulfil the needs mentioned above.

The present invention refers to compounds of the formula (I) 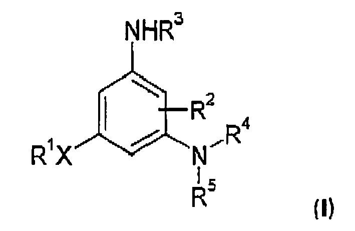 wherein
- X: is oxygen or sulphur;
- R¹: is (C₁-C₄)-alkyl, (C₁-C₄)-alkyl which is substituted by hydroxy, (C₁-C₄)-alkoxy, hydroxy-(C₁-C₄)-alkoxy, CN, -COOR⁶, -CON(R⁶)₂ or -N(R⁶)₂ or is phenyl;
- R²: is hydrogen, methyl or ethyl;
- R³: is hydrogen, (C₁-C₄)-alkylsulfonyl, (C₁-C₄)-alkylsulfonyl which is substituted by hydroxy, halogen, cyano, (C₁-C₄)-alkoxy or -N(R⁶)₂ or is -SO₂-CH = CH₂, -So₂N(R⁶)₂ or -PO(OR⁶)₂; or
- R³: is hydroxy-(C₁-C₄)-alkyl if R⁴ and R⁵ are not hydrogen;
- R⁴: is hydrogen, (C₁-C₄)-alkyl, (C₁-C₄)-alkyl which is substituted by hydroxy, (C₁-C₄)-alkoxy, or hydroxy-(C₁-C₄)-alkoxy;
- R⁵: is hydrogen, (C₁-C₄)-alkyl, (C₁-C₄)-afkyl which is substituted by hydroxy, (C₁-C₄)-alkoxy or hydroxy-(C₁-C₄)-alkoxy;
- R⁶: is hydrogen, (C₁-C₄)-alkyl or hydroxy-(C₁-C₄)-alkyl;
whereas R³, R⁴ and R⁵ are all hydrogen or are different and whereas the compound of the formula (I) wherein R¹ is methyl and R² to R⁵ are hydrogen is diesclaimt.

(C₁-C₄)-Alkyl groups may be straight-chain or branched and are for example methyl, ethyl, n-propyl, isopropyl, n-butyl, n-pentyl, isopentyl or n-hexyl. The same logic applies for alkoxy groups, which accordingly are for example methoxy and ethoxy.
Halogen is preferably fluorine, chlorine, bromine and iodine.

The compounds of the formula (I) can be present in form of their acid addition salts of organic or inorganic acids. Examples of such acid additions salts are cosmetically acceptable salts like chlorides, sulfates, phosphates, acetates, propionates, lactates and citrates.

In preferred compounds of formula (I) R² is preferably hydrogen or methyl.
In further preferred compounds of the formula (I) R³, R⁴ and R⁵ are all hydrogen.

Especially preferred compounds of this type are the compounds of formula (Ia) 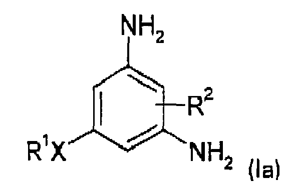 wherein
X is oxygen;
R¹ is phenyl, ethyl or methyl or ethyl substituted by hydroxy, -COOH, -CONH₂, cyano or dimethylamino;
R² is hydrogen or methyl;

Examples of especially preferred compounds of formula (la) are the compounds of formulae (Ib) to (Ij)

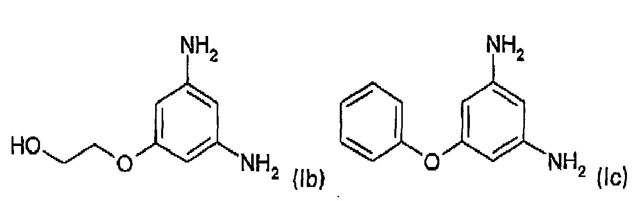

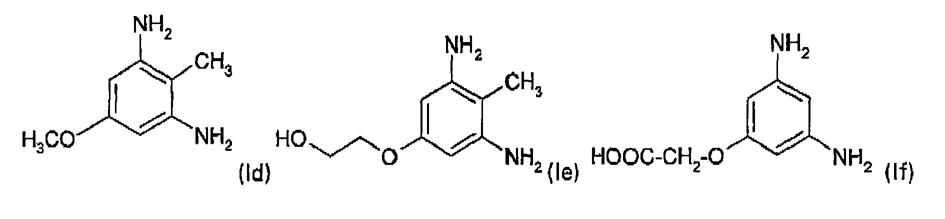

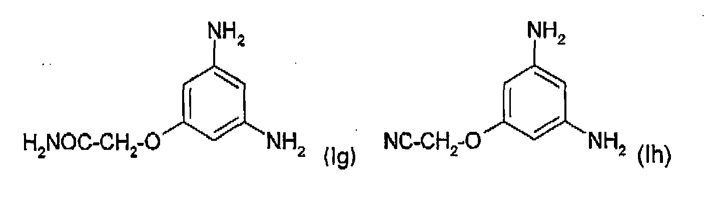

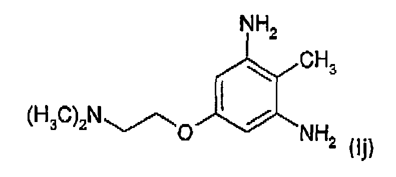

In further preferred compounds of the formula (I) at least one of R³, R⁴ and R⁵ is not hydrogen.
Especially preferred compounds of this type are compounds of formula (I), wherein
X is oxygen;
R¹ is phenyl, methyl, ethyl, hydroxymethyl or 2-hydroxy-ethyl, -COOH, -CONH₂, cyano or dimethylamino;
R² is hydrogen or methyl;
R³ is hydrogen, hydroxymethyl, 2-hydroxy-ethyl, methylsulfonyl or ethylsulfonyl which is unsubstituted or substituted by hydroxy, chlorine or -N(R⁵)₂ or is -SO₂-CH = CH₂, -PO(OR⁵)₂ or -SO₂N(R⁵)₂;
R⁴ is hydrogen, methyl, ethyl, hydroxymethyl or 2-hydroxy-ethyl;
R⁵ is hydrogen, methyl, ethyl, hydroxymethyl or 2-hydroxy-ethyl; and
each of R⁶, independently is hydrogen, methyl, ethyl or hydroxyethyl,
whereas at least one of R³, R⁴ and R⁵ is other than hydrogen.

In further especially preferred compounds of the formula (I) R³ is hydroxymethyl or 2-hydroxy-ethyl; R⁴ is methyl, ethyl, hydroxymethyl or 2-hydroxy-ethyl; and R⁵ is methyl, ethyl, or hydroxymethyl or 2-hydroxy-ethyl.

Examples of especially preferred compounds of this type are the compounds of formulae (Ik) to (Is)

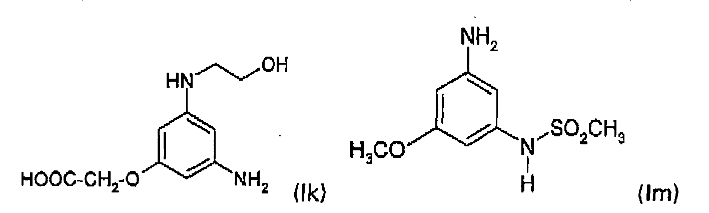

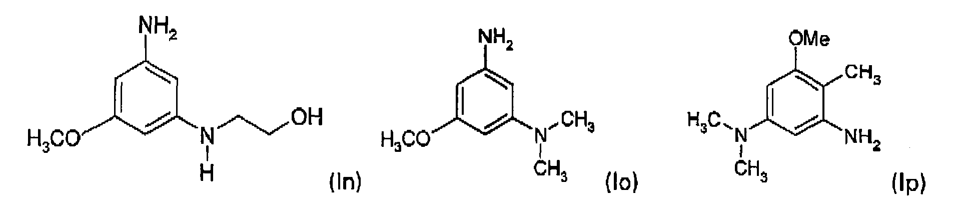

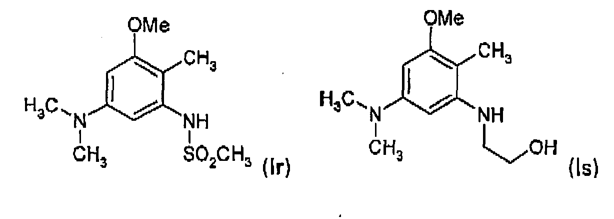

The inventive compounds of formula (I) can be prepared by methods which are in principle known to a person of ordinary skill in the art and which are described in literature, for example in the patent literature cited above.

For example, a compound of formula (I), wherein R² to R⁵ are hydrogen and X is oxygen, can be obtained by reducing a compound of formula (II)

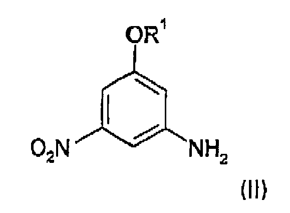

Reduction can be performed using known methods, for example with iron-II-sulfate as reducing agent.

Additional inventive compounds of formula (I) can be obtained by reacting a compound of formula (II) with reagents, which are capable to introduce R³, R⁴ or R⁵ into the molecule,
The compound of formula (II) can for example be reacted with alkylsulfonylchlorides or substituted alkylsulfonlychlorides like methylsulfonylchloride, chloromethylsulfonylohloride, 2-chloro-1-ethanesulfonylchloride or with sulfamoylamides like dimethylsulfamoylchloride or with dialkylchlorophosphates like diethylchlorophosphat. Subsequent reduction of the nitro group results in inventive compounds of formula (I) wherein R⁴ and R⁵ are hydrogen and R³ is other than hydrogen.
These compounds can finally be reacted with reagents which introduce R⁴ and/or R⁵ into the molecule, for example with chloroformic acid 2-chloroethylester, which introduces the -CH₂CH₂OH group- Accordingly, inventive compounds of formula (I) wherein neither R³, nor R⁴ and/or R⁵ are hydrogen are available. Alternatively, it is also possible to first introduce R⁴ and/or R⁵ into the compound of formula (II), followed by reduction. This leads to inventive compounds of the formula (I) wherein R³ is hydrogen and R⁴ and/or R⁵ are other than hydrogen.

The compounds of formula (II) can for example be obtained by introducing a protecting group, preferably the acetyl group, into 3-Amino-5-nitrophenol to protect the amino group and reacting the compound obtained with a reagent, which is capable to introduce R¹ into the molecule. Subsequent cleavage of the protecting group finally results in the compound of formula (II).
Reagents which are capable to introduce R¹ into the molecule are for example alkylhalogenids Y-R¹, wherein Y is preferably chlorine. An example is 2-chloroethanol, In case R¹ is methyl, dimethylsulfate is a preferred reagent.

An additional valuable starting compound for the preparation of inventive compounds of formula (I) is 3,5-diamino-4-methylphenol, which can be reacted with for example acetic anhydride to obtain N-(3-acetylamino-5-hydroxy-2-methyl)-acetamide, into which R¹ can be introduced as described above. Subsequent cleavage of the acetyl groups result in inventive compounds of the formula (laa) 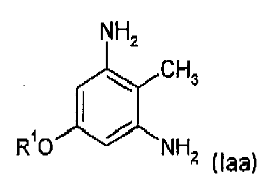 wherein R¹ is defined as given above.

A further valuable starting compound for the preparation of inventive compounds of formula (I) is 3-Methoxy-4-methyl-5-nitrophenylamine, which can be reacted with alkylating agents like alkylhalogenids Y-R⁴ or Y-R⁵, wherein Y is preferably chlorine and R⁴ and R⁵ are defined as given above. An example of Y-R⁴ or Y-R⁵ is chlorethanol. In case R⁴ and/or R⁵ are methyl, dimethylsulfate is a preferred reagent. After reduction of the nitro group further conversion with acylation reagents like methylsulfonyl-chloride or chloroformic acid 2-chloroethylester is possible. Preparation of 3-Methoxy-4-methyl-5-nitrophenylamine is described in Tetrahedron (1971), 27, page 1551ff.

The inventive compounds of formula (I) can advantageously be used as couplers which by reacting with developers form oxidation dyes for hair dyeing, especially human hair dyeing. Accordingly, the present invention also refers to hair dye compositions comprising at least one compound of the general formula (I).

The inventive hair dye compositions preferably comprise in addition to the compounds of the formula (I) as couplers one or more developers, which couplers and developers are capable to form an oxidation dye for hair dyeing.

Preferred developers are p-phenylenediamine derivatives such as benzene-1,4-diamine (commonly known as p-phenylenediamine), 2-mothyl-benzene-1,4-diamine, 2-chloro-benzene-1,4-diamine, N-phenyl-benzene-1,4-diamine, N-(2-ethoxyethyl)benzene-1,4-diamine, 2-[(4-amino-phenyl)-(2-hydroxy-ethyl)-amino]-ethanol (commonly known as N,N-bis(2-hydroxyethyl)-p-phenylenediamine) (2,5-diamino-phenyl)-methanol, 1-(2,5-diamino-phenyl)-ethanol, 2-(2,5-diaminophenyl)-ethanol, N-(4-aminophenyl)benzene-1,4-diamine, 2,6-dimethyl-benzene-1,4-diamine, 2-isapropyl-benzene-1,4-diamine, 1-[(4-aminophenyl)amino]-propan-2-ol, 2-propyl-benzene- 1 ,4-diamine, 1,3-bis[(4-aminophenyl)(2-hydroxyethyl)-amino]propan-2-ol, N⁴,N⁴,2-trimethylbenzene-1,4-diamine, 2-methoxy-benzene-1,4-diamine, 1-(2,5-diaminophenyl)ethane-1,2-diol, 2,3-dimethyl-benzene-1,4-diamine, N-(4-amino-3-hydroxy-phenyl)-acetamide, 2,6-diethylbenzene-1,4-diamine, 2,5-dimethylbenzene.1 ,4-diamine, 2-thien-2-ylbenzene-1,4-diamine, 2-thien-3-ylbenzene-1,4-diamine, 2-pyridin-3-ylbenzene-1,4-diamine, 1,1 -biphenyl-2,5-diamine, 2-(methoxymethyl)benzene-1,4-diamine, 2-(aminomethyl)benzene-1,4-diamine, 2-(2,5-diaminophenoxy)ethanol, N-[2-(2,5-diaminophenoxy)ethyl]-acetamide, N,N-dimethylbenzene-1,4-diamine, N,N-diethylbenzene-1,4-diamine, N,N-dipropylbenzene-1 ,4-diamine, 2-[(4-aminophenyl)(ethyl)amino]ethanol, 2-[(4-amino-3-methyl-phenyl)-(2-hydroxy-ethyl)-aminol-ethanol, N-(2-methoxyethyl)-benzene-1,4-diamine, 3-[(4-aminophenyl)amino]propan-1-ol, 3-[(4-aminophenyl)-amino]propane-1,2-diol, N-{4-[(4-aminophenyl)amino]butyl{benzene-1 ,4-diamine, and 2-[2-(2-}-2-[(2,5-diaminophenyl)-oxy]ethoxy}ethoxy}ethoxy)benzene-1,4-diamine;
p-aminophenol derivatives such as 4-amino-phenol (commonly known as p-aminophenol), 4-methylamino-phenol, 4-amino-3-methyl-phenol, 4-amino-2-hydroxymethyl-phenol, 4-amino-2-methyl-phenol, 4-amino-2-[(2-hydroxy-ethylamino)-methyl]-phenol, 4-amino-2-methoxymethyl- phenol, 5-amino-2-hydroxy-benzoic acid, 1-(5-amino-2-hydroxy-phenyl)-ethane-1,2-diol, 4-amino-2-(2 .hydroxy-ethyl)-phenol, 4-amino-3-(hydroxymethyl)phenol, 4-amino-3-fluorophenol, 4-amino-2-(aminomethyl)-phenol, and 4-amino-2-fluoro-phenol; o-aminophenol derivatives such as 2-amino-phenol (commonly known as o-aminophenol), 2,4-diaminophenol, 2-amino-5-methyl-phanol, 2-amino-6-methylphenol, N-(4-amino-3-hydroxy-phenyl)-acetamide, and 2-amino-4-methyl-phenol; and
heterocyclic derivatives such as pyrimidine-2,4,5,6-tetramine (commonly known as 2,4,5,6-tetraaminopyridine), 1 -methyl-1 H-pyrazole-4,5-diamine, 2-(4,5-diamino-1 H-pyrazol-1-yl)ethanol, N<2> ,N < 2 > -dimethyl-pyridine-2,5-diamine, 2-[(3-amino-6-methoxypyridin-2-yl)amino]ethanol, 6-methoxy-N < 2 > -methylpyridine-2,3-diamine, 2,5,6-triaminopyrimidin-4(1H)-one, pyridine-2,5-diamine, 1-isopropyl-1 H-pyrazole-4,5-diamine, 1-(4-methylbenzyl)-1 H-pyrazole-4,5-diamine, 1-(benzyl)-1 H-pyrazole-4,5-diamine, 1-(4-chlorobenzyl)-1 H-pyrazole-4,5-diamine and 2,5,6-triamino-4-pyrimidinol sulfate.

The hair dye compositions according to the present invention can in addition to the compounds of the formula (I) comprise further couplers. Such further couplers are preferred couplers as normally used to produce oxidation dyes for hair dyeing.
Examples of such couplers are phenols, resorcinol and naphthol derivatives such as naphthalene-1,7-diol, benzene-1,3-diol, 4-chlorobenzene-1,3-diol, naphthalen-1-ol, 2-methyl-naphthalen-1-ol, naphthalene-1 ,5-diol, naphthalene-2,7-diol, benzene-1,4-diol; 2-methyl-benzene-1,3-diol, 7-amino-4-hydroxy-naphthalene-2-sulfonic acid, 2-isopropyl-5-methylphenol, 1,2,3,4-tetrahydro-naphthalene-1,5-diol, 2-chloro-benzene-1,3-diol, 4-hydroxy-naphthalene-1-sulfonic acid, benzene-1,2,3-triol, naphthalene-2,3-diol, 5,dichloro-2-methylbenzene-1,3-diol, 4,6-dichlorobenzene-1,3-diol, and 2,3-dihydroxy-[1,4]naphthoquinone; m-phenylenediamines such as 2,4-diaminophenol, benzene- 1,3-diamine, 2-(2,4-diamino-phenoxy)-ethanol, 2-[(3-amino-phenyl)-(2-hydroxyethyl)-amino]-ethanol, 2-mehyl-benzene-1,3-diamine, 2-[[2-(2,4-diamino-phenoxy)-ethyl]-(2-hydroxy-ethyl)-amino]-ethanol, 4-{3-[(2,4-diaminophenyl)oxy]propoxy}benzene-1,3-diamine, 2-(2,4-diamino-phenyl)-ethanol, 2-(3-amino-4-methoxy-phenylamino)-ethanol, 4-(2-amino-ethoxy)-benzene-1,3-diamine, (2,4-diamino-phenoxy)-acetic acid, 2-[2,4-diamino-5-(2-hydroxy-ethoxy)-phenoxy]-ethanol, 4-ethoxy-6-methylbenzene-1,3-diamine, 2-(2,4-diamino-5-methyl-phenoxy)-ethanol, 4,6-dimethoxybenzene-1,3-diamine, 2-[3-(2-hydroxy-ethylamino)-2-methyl-phenylamino]-ethanol, 3-(2,4-diamino-phenoxy)-propan-1-ol, N-[3-(dimethylamino)phenyl]urea, 4-methoxy-6-methylbenzene-1,3-diamine, 4-fluoro-6-methylbenzene-1,3-diamine, 2-({3-[(2-hydroxyethyl)amino]-4,6-dimethoxyphenyl}-amino)ethanol, 3-(2,4-diaminophenoxy)-propane-1,2-diol, 2-[2-amino4-(methylamino)-phenoxy]ethanol, 2-[(5-amino-2-ethoxy-phenyl)-(2-hydroxy-ethyl)-amino]-ethanol, 2-[(3-aminophenyl)amino]ethanol, N-(2-aminoethyl)benzene-1,3-diamine, 4{[(2,4-diamino-phenyl)oxy]methoxy}-benzene-1,3-diamine, and 2,4-dimethoxybenzene-1,3-diamine;
m-aminophenols such as 3-amino-phenol, 2-(3-hydroxy-4-methyl-phenylamino)-acetamide, 2-(3-hydroxy-phenylamino)-acetamide, 5-amino-2-methyl-phenol, 5-(2-hydroxy-ethylamino)-2-methyl-phenol, 5-amino-2,4-dichloro-phenol, 3-amino-2-methyl-phenol, 3-amino-2-chloro-6-methyl-phenol, 5-amino-2-(2-hydroxy-ethoxy)-phenol, 2-chloro-5-(2,2,2-trifluoro-ethylamino)-phenol, 5-amino-4-chloro-2-methyl-phenol, 3-cyclopentylamino-phenol, 5-[(2-hydroxyethyl)amino]4-methoxy-2-methylphenol, 5-amino-4-methoxy-2-methylphenol, 3-(dimethylamino)phenol, 3-(diethylamino)phenol, 5-amino4-fluoro-2-methylphenol, 5-amino-4-ethoxy-2-methylphenol, 3-amino-2,4-dichloro-phenol, 3-[(2-methoxyethyl)amino]phenol, 3-[(2-hydroxyethyl)amino]phenol, 5-amino-2-ethylphenol, 5-amino-2-methoxyphenol, 5-[(3-hydroxypropyl)amino]-2-methylphenol 3-[(3-hydroxy-2-methylphenyl)-amino]propane-1,2-diol, and 3-[(2-hydroxyethyl)amino]-2-methylphenol; and
heterocyclic derivatives such as; 3,4-dihydro-2H-1,4-benzoxazin-6-ol, 4-methyl-2-phenyl-2,4-dihydro-3H-pyrazol-3-one, 6-methoxyquinolin-8-amine, 4-methylpyridine-2,6-diol, 2,3-dihydro-1,4-benzodioxin-5-ol, 1,3-benzodioxol-5-ol, 2-(1,3-benzodioxol-5-ylamino)ethanol, 3,4-dimethylpyridine-2,6-diol, 5-chloropyridine-2,3-diol, 2,6-dimethoxypyridine-3,5-diamine, 1,3-benzodioxol-5-amine, 2-{(3,5-diamino-6-(2-hydroxy-ethoxy)-pyridin-2-yl]oxy}-ethanol, 1 H-indol-4-ol, 5-amino-2,6-dimethoxypyridin-3-ol, 1 H-indole-5,6-diol, I H-indol-7-ol, 1 H-indol-5-ol, 1 H-indol-6-ol, 6-bromo-1,3-benzodioxol-5-ol, 2-aminopyridin-3-ol, pyridine-2,6-diamine, 3-[(3,5-diaminopyridin-2-yl)oxy]propane-1,2-diol, 5-[(3,5-diaminopyridin-2-yl)oxy]pentane-1,3-diol, 1 H-indole-2,3-dione, indoline-5,6-diol, 3,5-dimethoxypyridine-2,6-diamine, 6-methoxypyridine-2,3-diamine, and 3,4-dihydro-2H-1,4-benzoxazin-6-amine.
The inventive hair dye compositions usually contain couplers at a total concentration of 0.001 to 10%, preferably 0.005 to 7.5% and more preferably 0.01 to 5% by weight, based on the weight of the hair dyeing composition and developers at a total concentration of 0.001 to 10%, preferably 0.005 to 7.5 % and more preferably 0.01 to 5% by weight, calculated based on the weight of the hair dyeing composition.

The hair dye compositions according to the present invention can additionally comprise at least one direct dye. Such direct dyes can be cationic, anionic or neutral and can be of natural or synthetic origin. Preferred natural dyes are plant dyestuffs. Suitable dyes of such types are known on the market for hair colouring applications.

Suitable cationic dyestuffs are for example Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57, Basic Orange 31 and Basic Yellow 87.
Additional examples are the dyes disclosed in WO 95/15144 which are included herein by reference.
The hair dye compositions according to the present invention can contain cationic dyestuffs at a concentration of 0.001 to 2%, preferably 0.005 to 1 ,5% and more preferably 0.01 to 1 % by weight, based on the weight of the hair dyeing composition.

Suitable anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium and potassium.
The hair dye compositions according to the present invention can contain anionic dyestuffs at a concentration of 0.001 to 2%, preferably 0.005 to 1.5% and more preferably 0.01 to 1 % by weight, based on the weight of the hair dyeing composition.

Suitable neutral dyes (HC dyes or nitro dyes) are HC Blue No.2, HC Blue No.4, HC Blue No.5, HC Blue No.6, HC Blue No.7, HC Blue No.8, HC Blue No.9, HC Blue No. 10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.1 0, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No,11, HC Yellow No.12, HC Yellow No.l3, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.
The hair dye compositions according to the present invention can contain neutral dyestuffs at a concentration of 0.001 to 2%, preferably 0.01 to 1.5% and more preferably 0.05 to 1 % by weight, based on the weight of the hair dyeing composition.

Suitable plant dyestuffs are henna (red or black), alkanna root, laccaic acid, indigo, logwood powder, madder root and rhubarb powder.
The pH of the hair dye compositions according to the present invention may range from 3 to 12, preferably from 5 to 11. The pH might be adjusted to the desirable value by the use of acidifying and alkalising agents.
Suitable acidifying agents are for example mineral and organic acids, such as hydrochloric acid, orthophosphoric acid, sulfuric acid, carboxylic acids such as acetic acid, tartaric acid, citric acid, and lactic acid, and sulphonic acids.
Suitable alkalizing agents are for example aqueous ammonia, alkali metal carbonates, alkalonamine such as monoethanolamine, diethanolamine, and triethanolamine, and derivatives thereof, sodium hydroxide.

In addition to the above-described components, those ordinarily employed as a raw material for cosmetics can be added to the hair dye compositions of the present invention. Examples of such an optional component include hydrocarbons such as squalane; animal or vegetable fats and oils; higher fatty acids such as oleic acid; organic solvents such as 1,2-propylene glycol; penetration promoters; cationic surfactants; natural or synthetic polymers; higher alcohols such as cetearyl alcohol; ethers; amphoteric surfactants; nonionic surfactants such as stearoyl monoethanolamide, coconut monoethanolamide, propylene glycol monostearate, polyoxyethylene (5) coconut amide, anionic surfactants such as sodium cetyl sulfate, sodium stearyl sulfate, potassium stearate, sodium stearate, polyoxyethylene (5) oleyl ether phosphate; protein derivatives; active ingredients such as coenzyme Q10; amino acids; antiseptics; chelating agents; stabilizers; antioxidants; plant extracts; crude drug extracts; vitamins such as retinyl palmitate; colorants; perfumes; catalysts such as potassium iodide; and ultraviolet absorbers.

The hair dye compositions of the present invention can be prepared in a conventional manner by mixing the components in the required amounts.

In order to use the hair dye compositions of the present invention to dye hair they have to be mixed with an oxidation agent, which allows the formation of an oxidation dye by reaction of the coupler component with the developer component and which is usually called developer composition.
Preferred oxidation agents are peroxide containing agents, particularily hydrogen peroxide, or precursors thereof. Also suitable are urea peroxide, sodium perborate, sodium percarbonate, melamine peroxide, and persulfates such as ammonium persulfate, sodium persulfate and potassium persulfate. Oxygen can also be used as oxidation agent.
Typically, hydrogen peroxide or its addition compounds with urea, melamine, sodium borate or sodium carbonate are used in the form of a 1 % to 20%, preferably 2% to 15 %, most preferably 2% to 12% preparation.

The hair dye composition according to the present invention as well as the oxidation agent can be present in the form of powder, transparent liquid, emulsion, cream, gel, paste, aerosol, aerosol foam or the like.

The present application also refers to a hair dyeing kit which comprises in separate containers a hair dye composition according to the present invention, a developer composition and optionally further components.
Such further components are for example a shampoo, a conditioning composition, a hair treatment product, gloves and instructions for use.
Usually, the hair dye composition according to the present invention and the developer composition (oxidation agent) are mixed just before hair dyeing and the mixture is applied to the hair in a sufficient amount, which depends on the hair abundance, generally from about 60 to 200 grams.
The mixture is then allowed to act on the hair for about 10 to about 45 minutes, preferably about 30 minutes, at about 15 to 50°C. Thereafter, the hair is rinsed with water and dried. If necessary, it is washed with a shampoo. Subsequently the hair is dried.

The hair dye composition according to the present invention provides hair dyeing which has good uptake, good selectivity, as well as good stability to light and shampooing with improved brilliance.

### Example 1: 2-(3,5-Diamino-phenoxy)-ethanol (compound of formula (lb))

### a) N-(3-Hydroxy-5-nitrophenyl)-acetamide

15,4 g of 3-Amino-5-nitrophenol were suspended in 50 ml of water. During subsequent addition of 19 ml of acetic acid anhydride within 15 minutes temperature raised to 60°C. Then the reaction mixture was heated to 80°C and kept there for 3 hours. After cooling the separated product was isolated by filtration and dried.
Yield: 19,04 g (97,1%)

### b) N-(3-(2-Hydroxyethoxy)-5-nitrophenyl)-acetamide

5,47 g of N-(3-Hydroxy-5-nitrophenyl)-acetamide were dissolved in 17 ml of water and 7 ml of 5n NaOH. After addition of 1,9 ml of 2-Chloroethanol the reaction mixture was heated to 85°C and kept there for 7 hours: Thereafter additional 1,4 ml of 5n NaOH and 0,4 ml of 2-Chloroethanol were added and the mixture kept for further 4 hours at 85°C. After cooling the separated product was isolated by filtration and dried.
Yield: 6,11 g(90,8%)

### c) 2-(3-Amino-5-nitro-phenoxy)-ethanol

6 g of N-(3-(2-Hydroxyethoxy)-5-nitrophenyt)-acetamide were suspended in 45 ml of HCI 15 % and heated to 100°C for 1 hour. After cooling of the reaction mixture the product crystallizes as hydrochloride. It was isolated by filtration and dried.
Yield: 5,0 g (85,2%)

### d) 2-(3,5-Diamino-phenoxy)-ethanol

39,03 g of iron-II-sulfate heptahydrate were dissolved in 200 ml of water and heated under nitrogen to 80°C. 4,69 g of 2-(3-Amino-5-nitro-phenoxy)-ethanol x HCI were dissolved in 40 ml of water and dropped into the hot ironsulfate solution. Within 30 minutes 50,7 ml of ammonium hydroxide, 26% were added portionwise and the reaction temperature subsequently kept between 85 and 90°C for 1 hour. Then the hot reaction mixture was filtered and after cooling extracted with n-butanol: After drying of the organic layer and evaporation of the solvent the product was received as solid material.
Yield: 1,9 g (56,4%)

### Example 2: N-(3-Amino-5-methoxyphenyl)-methansulfonamide (compound of formula (lm))

### a) N-(3-Methoxy-5-nitrophenyl)-acetamide

9,8 g of N-(3-Hydroxy-5-nitrophenyl)-acetamide (see example 1 a)) were dissolved in 30 ml of water and 12,5 ml of 5n NaOH. During subsequent addition of 5,7 ml of dimethylsulfate temperature raised to 40°C. Then the reaction mixture was heated to 60°C and kept there for 3 hours. Thereafter additional 3,1 ml of 5n NaOH and 1,5 ml of dimethylsulfate were added and the mixture kept for further 3 hours at 60°C. After cooling the separated product was isolated by filtration.
Yield: 26 g as wet filter cake

### b) 3-Methoxy-5-nitrophenylamine

26 g of wet N-(3-Methoxy-5-nitrophenyl)-acetamide were suspended in 90 ml of HCl 15 % and heated to 100°C for 1 hour. After cooling the reaction mixture was neutrallized with NaOH 33 %. The separated product was isolated by filtration and dried.
Yield: 7,51 g (89,3%)

### c) N-(3-Methoxy-5-nitrophenyl)-methansulfonamide

2,0 g of 3-Methoxy-5-nitrophenylamine were suspended in 20 ml of acetonitrile. After addition of 1,1 ml of pyridine, 1,0 ml of methylsulfonylchloride, dissolved in 3 ml of acetonitrile, were introduced dropwise. Temperature of the reaction mixture raised to 30°C. After addition of sulfonylchloride, the reaction mixture was heated to 60°C and kept there until no 3-Methoxy-5-nitrophenylamine was detectable. Solvent was removed by evaporation and the residue was taken up with water. The product was isolated by filtration and dried.
Yield: 2,9 g (99,0 %)

### d) N-(3-Amino-5-methoxyphenyl)-methansulfonamide

22,13 g of iron-II-sulfate heptahydrate were dissolved in 120 ml of water and heated under nitrogen to 80°C. 2,8 g of N-(3-Methoxy-5-nitrophenyl)-methansulfonamide were dissolved in 30 ml of hot ethanol and introduced into the hot ironsulfate solution. Within 30 minutes 31 ml of ammonium hydroxide, 26% was added portionwise and the reaction temperature was kept between 85 and 90°C for 1 hour. Then the hot reaction mixture was filtered and after cooling extracted with ethylacetate, After drying of the organic layer and evaporation of the solvent the product was received as solid material.
Yield: 2,17 g (88,2%)

### Example 3: 2-(3-Amino-5-methoxy-phenylamino)-ethanol (compound of formula (In))

### a) 2-(3-Methoxy-5-nitrophenylamino)-ethanol

2,25 g of 3-Methoxy-5-nitrophenylamine (see Example 2b)) were dissolved in 20 ml of 1,2-dimethoxyethane and 3 ml of water. Then 805 mg of calcium carbonate were added and the mixture was heated to 80°C. 1,5 ml of chloroformic acid 2-chloroethyl-ester were added dropwise and thereafter the reaction mixture was kept at 80°C for 1 hour, The reaction mixture was diluted with 2ml of 1,2-dimethoxyethane 5 ml of water and 5 g of potassium hydroxide solution, 50%. This mixture was heated again to 80°C and kept there for 3 hours. Then 1,2-dimethoxyethane was removed by evaporation and the separated product isolated by filtration and dried.
Yield: 2,61 g (92,0%)

### b) 2-(3-Amino-5-methoxy-phenylamino)-ethanol

23,42 g of iron-ll-sulfate heptahydrate were dissolved in 120 ml of water and heated under nitrogen to 80°C. 2,55 g of 2-(3-Methoxy-5-nitrophenylamino)-ethanol were dissolved in 50 ml of hot ethanol and introduced into the hot ironsulfate solution. Within 15 minutes 30,4 ml of ammonium hydroxide 26% were added portionwise. The reaction temperature was kept between 85 and 90°C for 1 hour. Then the hot reaction mixture was filtered and after cooling extracted with n-butanol. After drying of the organic layer and evaporation of the solvent the product was received as viscous oil.
Yield: 2,12 g (93,9%)
The oil was dissolved in 4 ml methanol and mixed under ice cooling with 0,7 ml sulphuric acid. The product crystallizes and was isolated by filtration.
Yield: 2,95 g (93,4%)

### Example 4: 5-Methoxy-2-methyl-benzene-1,3-diamine (compound of formula (Id))

### a) N-(3-Acetylamino-5-hydroxy-2-methyl-phenyl)-acetamide

13,82 g of 3,5-Diamino-4-methylphonol were suspended in 50 ml of water. During subsequent addition of 38 ml of acetic acid anhydride within 30 minutes temperature raised to 60°C. Then the reaction mixture was heated to 90°C and kept there for 4 hours. After cooling the separated product was isolated by filtration and dried.
Yield: 13,91 g (62,6%)

### b) N-(3-Acetylamino-5-methoxy-2-methyl-phenyl)-acetamide

5,56 g of N-(3-Acetylamino-5-hydroxy-2-mothyl-phenyl)-acetemide were dissolved in 20 ml of water and 7,5 ml of 5n NaOH. During addition of 3,4 ml of dimethylsulfate temperature raised to 40°C. Then the reaction mixture was heated to 60°C and kept there for 6 hours. After cooling the separated product was isolated by filtration and dried.
Yield: 4,67 g (79,0%)

### c) 5-Methoxy-2-methyl-benzene-1,3-diamine

4,59 g of N-(3-Acetylamino-5-methoxy-2-methyl-phenyl)-acetamide were stirred in 25 ml HCI 15% for 1 hour at 90°C. After cooling the reaction mixture was neutrallized with dilute NaOH solution and the product was extracted with n-butanol. After drying of the organic layer and evaporation of n-butanol the product was received as tacky material.
Yield: 2,36 g (79,8%)

### Example 5: 2-(3,5-Diamino-4-methyl-phenoxy)-ethanol (compound of formula (le))

### a) N-(3-Acetylamino-5-(2-hydroxyethoxy)-2-methyl-phenyl)-acetamide

4,45 g of N-(3-Acetylamino-5-hydroxy-2-methyl-phenyl)-acetemide (see Example 5a)) were dissolved in 15 ml of water and 6 ml of 5n NaOH. After addition of 1,9 ml of 2-chloroethanol the reaction mixture was heated to 90°C and kept there for 3 hours. During reaction additional 10 ml of water were added to keep the mixture liquid. After cooling the separated product was isolated by filtration and dried.
Yield: 4,19 g (78,6%)

### b) 2-(3,5-Diamino-4-methyl-phenoxy)-othanol

2,1 g of N-(3-Acetylamino-5-(2-hydroxyethoxy)-2-methyl-phenyl)-acetamide were dissolved in 15 ml methanolic hydrogen chloride (5 moles HCI / I methanole) and heated for 2 hours to 60-65 °C. The product was isolated as solid after evaporation to dryness.
Yield: 1,93 g (95,8%)

### Example 6: (3,5-Diamino-phenoxy)-acetic acid (compound of formula (If)) a)(3-Acetylamino-5-nitro-phenoxy)-acetic acid ethylester

A mixture of 2,94 g of 3-Acetylamino-5-nitro-phenol, 2,07 g of potassium carbonate and 1,37 g of potassium iodide was suspended in 25 ml acetone. After addition of 2,02 g of chloroacetic acidethylester temperature was raised to 60°C and kept there until no 3-Acetylamino-5-nitro-phenol was detectable. Solvent was removed by evaporation and the residue was taken up with water; the undissolved product was isolated by filtration and dried.
Yield: 3,94 g (93,1 %)

### b) (3-Amino-5-nitro-phenoxy)-acetic acid

3,94 g of (3-Acetylamino-5-nitro-phenoxy)-acetic acid ethylester was suspended in 20 ml NaOH, 10 % and heated for 1 hour to 60°C. After cooling pH 4,5 was set with diluted HCI. The separated product was isolated by filtration and dried.
Yield: 2,93 g (98,8 %)

### c)(3,5-Diamino-phenoxy)-acetic acid

26,86 g of iron-II-sulfate heptahydrate were dissolved in 125 ml of water and heated under nitrogen to 80°C. 2,93 g of (3-Amino-5-nitro-phenoxy)-acetic acid were dissol-ved in 40 ml of hot ethanol-water mixture and introduced into the hot ironsulfate solution. Within 15 minutes 35 ml of ammonium hydroxide, 26% were added portion-wise. The reaction temperature was kept between 85 and 90°C for 1 hour. Then the hot reaction mixture was filtered and after evaporation of the ethanol and cooling the crystallized product was isolated by filtration and dried.
Yield: 2,05 g (81,5 %)

### Example 7: (3,5-Diamino-phenoxy)-acetamide (compound of formula (lg))

The procedure of example 7 was repeated with the exception that 1,54 g of chloroacetamide were used instead of 2,02 g of chloroacetic acidethylester. Yield: 1,74 g (64,2 % over all steps)

### Example 8; (3,5-Diamino-phenoxy)-acetonitrile (compound of formula (lh))

The procedure of example 7 was repeated with the exception that 1,25 g of chloroacetonitrile were used instead of 2,02 g of chloroacetic acidethylester. Yield: 1,57 g (63,9 % over all steps)

### Example 9; 5-(2-Dimethylaminoethoxy)-benzene-1,3-diamine (compound of formula (Ij))

The procedure of example 7 was repeated with the exception that 2,38 g of dimethyl-aminoothylchloride hydrochloride were used instead of 2,02 g of chloroacetic acid-ethylester.
Yield: 1,35 g (46 % over all steps)

### Example 10: (3-Amino-5-(2-hydroxyethylamlno)-phehoxy)-acetic acid (compound of formula (lk))

### a) (3-(2-Hydroxyethylamino)-5-nitro-phenoxy)-acetic acid

The procedure of example 3a was repeated with the exception that 2,93 g of (3-Ami-no-5-nitro-phenoxy)-acetic acid (see Example 6b)) were used instead of 2,25 g of 3-Methoxy-5-nitrophenylamine.
Yield: 3,07 g (87 %)

### b) (3-Amino-5-(2-hydroxyethylamino)-phenoxy)-acetic acid

The procedure of example 3b was repeated with the exception that 3,07 g of (3-(2-Hydroxyethylamino)-5-nitro-phenoxy)-acetic acid were used instead of 2,55 g of 2-(3-Methoxy-5-nitrophenylamino)-ethanol. The product crystallizes after evaporation of n-butanol.
Yield: 2,28 g (84,2%)

### Example 11: 5-Methoxy-N,N-dimethyl-benzene-1,3-diamine (compound of formula (lo))

### a) (3-Mothoxy-5-nitro-phenyl)-dimethylamine

4,2 g of 3-Methoxy-5-nitrophenylamine (see Example 2b)) were dissolved in 50 ml of chlorobenzene and after addition of 5 ml dimethylsulfate the reaction mixture was heated to115°C and kept there for 48 hours. Chlorobenzene was removed by evaporation and the residue was taken up with water; after neutralization with NaOH the product was isolated by filtration and dried.
Yield: 3,12 g (63,6%)

### b) 5-Methoxy-N,N-dimethyl-benzene-1,3-diamine

The procedure of example 1 d was repeated with the exception that 3,12g of (3-Methoxy-5-nitro-phenyl)-dimethylamine were converted with 31,11 g iron-II-sulfate heptahydrate. After filtration of the reaction mixture the product was extracted with n-butanol. After evaporation of n-butanol the oily residue crystallizes after addition of methanolic hydrogen chloride (5 moles HCI / l methanole).
Yield: 2,72 g (71,5%)

### Example 12: 5-Methoxy-4,N*1,N*1-trimethyl-benzene-1,3-diamine (compound of formula (lp))

### a) (3-Methoxy-4-methyl-5-nitro-phenyl)-dimethylamine

The procedure of example 11 a was repeated with the exception that 13,65 g of 3-Meth-oxy-4-methyl-5-nitrophenylamine were used.
Yield: 10,95 g (69,4%)

### b) 5-Methoxy-4,N *1,N*1-trimethyl-benzene-1,3-diamine

The procedure of example 11 b was repeated with the exception that 10,95 g of (3-Methoxy-4-methyl-5-nitro-phenyl)-dimethylamine were converted with 101,36 g iron-II-sulfate heptahydrate.
Yield: 11,24 g (85,4%)

### Example 13: N-(5-Dimethylamino-3-methoxy-2-methyl-phenyl)-methansulfon-amide (compound of formula (1r))

The procedure of example 2c was repeated with the exception that 3,8 g of 5-Methoxy-4,N*1,N*1-trimethyl-benzene-1,3-diamine were used.
Yield: 3,58g (92,3%)

### Example 14: 2-(5-Dimethylamino-3-methoxy-2-methyl-phenylamino)-ethanol (compound of formula (Is))

The procedure of example 3a was repeated with the exception that 3,8 g of 5-Methoxy-4,N*1,N*1-trimethyl-benzene-1,3-diamine were used
Yield: 2,94 g (87,5%)

### Application Example

The following Dye Compositions were produced:

| | |
|---|---|
| iso-propanol | 5.0 % |
| ammonia 25% active | 5.0 % |
| sodium sulfite | 1.0% |
| hydrogen peroxide | 3,0 % |
| developer I or 11 (see below) coupler (inventive compound; | 0.4 % |
| see below) | 0.4 % |
| water | up to 100 % |

The following developers werde used:

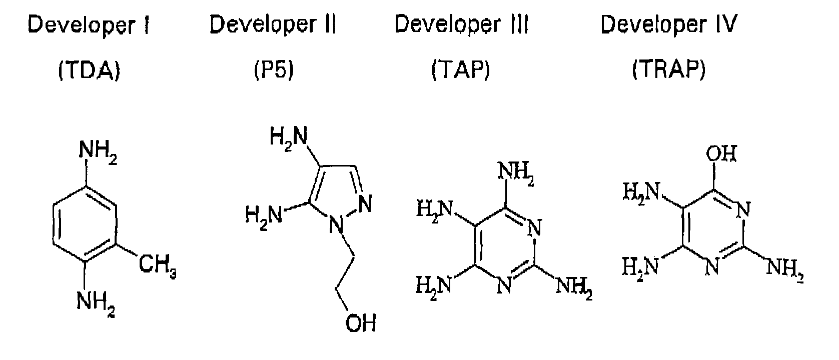

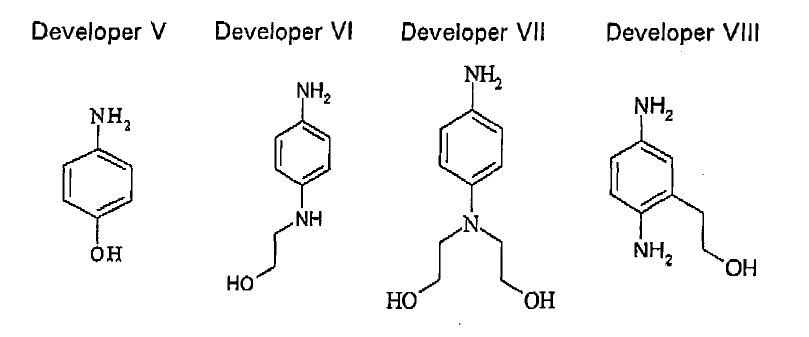

The Dye Compositions mentioned above were applied to undamaged white goat hair in amounts of approximately 1 g per g of hair at 50°C for 15min. or at 30°C for 30min. At the end of the processing time the tresses were rinsed with water, shampooed and then dried.

Brilliant colourings as given in the following Tables 1 and 2 were obtained.

**Table 1**

| Compound of the formula (I) | Developer I | Developer II |
|---|---|---|
| Ib | deep blue | intense yellowish red |
| Ic | blue | red |
| Id | deep blue | intense red |
| Ie | deep blue | intense red |
| Im | steel blue | blueish red |
| In | blue | intense red |
| If | blue | pink |
| Ig | deep blue | intense yellowish red |
| Ih | greenish blue | violet |
| Ij | dark blue | intense red |
| Ik | dark blue | Intense red |
| Io | blue | red |
| Ip | dark blue | intense red |
| Ir | blue | intense pink |
| Is | deep blue | intense red |

**Table 2**

| Developer | Compound of the formula (In) | Compound of the formula (ls) |
|---|---|---|
| I | blue | deep blue |
| II | intense red | intense red |
| III | orange | green |
| IV | brown | brownish |
| V | red brown | red brown |
| VI | black blue | deep blue |
| VII | violet | violet |
| VIII | blue | blue |

## Claims

1. Compound of the formula (I) 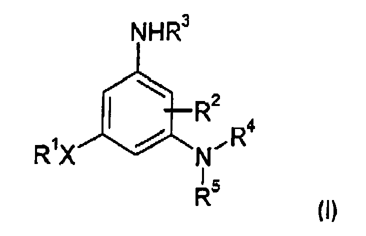 wherein
X is oxygen or sulphur;
R¹ is (C₁-C₄)-alkyl, (C₁-C₄)-alkyl which is substituted by hydroxy, (C₁-C₄)-alkoxy, hydroxy-(C₁-C₄)-alkoxy, CN, -COOR⁶, -CON(R⁶)₂ or -N(R⁶)₂ or is phenyl;
R² is hydrogen, methyl or ethyl;
R³ is hydrogen, (C₁-C₄)-alkylsulfonyl, (C₁-C₄)-alkylsulfonyl which is substituted by hydroxy, halogen, cyano, (C₁-C₄)-alkoxy or -N(R⁶)₂ or is -S0₂-CH = CH₂, -S0₂N(R⁶)₂ or -PO(OR⁶)₂; or
R³ is hydroxy-(C₁-C₄)-alkyl if R⁴ and R⁵ are not hydrogen;
R⁴ is hydrogen, (C₁-C₄)-alkyl, (C₁-C₄)-alkyl which is substituted by hydroxy, (C₁-C₄)-alkoxy, or hydroxy-(C₁-C₄)-alkoxy;
R⁵ is hydrogen, (C₁-C₄)-alkyl, (C₁-C₄)-alkyl which is substituted by hydroxy, (C₁-C₄)-alkoxy or hydroxy-(C₁-C₄)-alkoxy;
R⁶ is hydrogen, (C₁-C₄)-alkyl or hydroxy-(C₁-C₄)-alkyl;
whereas R³, R⁴ and R⁵ are all hydrogen or are different and whereas the compound of the formula (I) wherein R¹ is methyl and R² to R⁵ are hydrogen is diesclaimt.

2. Compound according to claim 1, wherein R² is hydrogen or methyl.

3. Compound according to claim 1 and/or 2 wherein R³, R⁴ and R⁵ are all hydrogen.

4. Compound according to claim 3, which is of the formula (Ia) 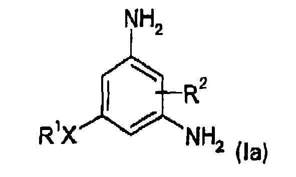 wherein
X is oxygen;
R¹ is phenyl, methyl, ethyl or methyl or ethyl substituted by hydroxy, -COOH, -CONH₂, cyano or dimethylamino;
R² is hydrogen or methyl;

5. Compound according to claim 1 and/or 2, wherein at least one of R³, R⁴ and R⁵ is not hydrogen.

6. Compound according to claim 5, which is of the formula (I) wherein
X is oxygen;
R¹ is phenyl, methyl, ethyl, hydroxymethyl or 2-hydroxy-ethyl, -COOH, -CONH₂, cyano or dimethylamino;
R² is hydrogen or methyl;
R³ is hydrogen, hydroxymethyl, 2-hydroxy-ethyl, methylsulfonyl or ethylsulfonyl which is unsubstituted or substituted by hydroxy, chlorine or -N(R⁶)₂ or is -SO₂-CH =CH₂, -PO(OR⁶)₂ or -SO₂N(R⁶)₂;
R⁴ is hydrogen, methyl, ethyl, hydroxymethyl or 2-hydroxy-ethyl;
R⁵ is hydrogen, methyl, ethyl, hydroxymethyl or 2-hydroxy-ethyl; and
R⁶ is hydrogen, methyl, ethyl or hydroxyethyl,
whereas at least one of R³, R⁴ and R⁵ is other than hydrogen.

7. Compound according to claim 6, wherein
R³ is hydroxymethyl or 2-hydroxy-ethyl;
R⁴ is methyl, ethyl, hydroxymethyl or 2-hydroxy-ethyl; and
R⁵ is methyl, ethyl, or hydroxymethyl or 2-hydroxy-ethyl.

8. Hair dye composition comprising one or more compounds of the formula (I) according to one or more of claims 1 to 7.

9. Hair dye composition according to claim 8, which comprises in addition to the compounds of the formula (I) as couplers one or more developers, which couplers and developers are capable to form an oxidation dye for hair dyeing.

10. Hair dye composition according to claims 9 and/or 10, which comprises in addition to the compounds of the formula (I) further couplers.

11. Hair dye composition according to one or more of claims 8 to 10, which comprises at least one direct dye.

12. Hair dyeing kit which comprises in separate containers a hair dye composition according to one or more of claims 8 to 11, a developer composition and optionally further components.
